# EUROPEAN PATENT APPLICATION

(11) **EP 4 454 739 A1**
(43) Date of publication of application: **30.10.2024**
(21) Application number: 23170819.9
(22) Date of filing: 28.04.2023
(51) Int. Cl.: B01D 63/08, C12M 3/06, B01D 69/10, B01D 25/26

(54) **FILTRATION DEVICE, IN PARTICULAR SMALL-SCALE FILTRATION DEVICE FOR PRODUCTION OF BIOPHARMACEUTICALS**

(71) Applicant: Sartorius Stedim Biotech GmbH, 37079 Göttingen (DE)
(72) Inventor: FRIESE, Thomas, 99752 Bleicherode (DE); GRELL, Gerid, 37075 Göttingen (DE); RÜSCH, Natalie, 37115 Duderstadt (DE); DORFSCHÄFER, Philipp, 34576 Homberg (DE); LÖWE, Thomas, 37077 Göttingen (DE)
(74) Representative: Prinz & Partner mbB

(57) **Abstract**

A filtration device (10), in particular a small-scale filtration device for production of biopharmaceuticals, comprises a housing including a top cover (12) having an unfiltrate inlet (18) and a bottom cover (14) having a filtrate outlet (22), and one or more filter supports (16) stacked between the top cover (12) and the bottom cover (14) along a vertical direction such that an upper side of each filter support (16) faces the top cover (12) and a lower side of each filter support (16) faces the bottom cover (14). Each filter support (16) is equipped with a flat upper filter (40) and a flat lower filter (42). Each filter (40, 42) has a filtrate side and an opposite unfiltrate side. The upper and lower filters (40, 42) are sealed to the upper and lower sides of the filter support (16), respectively, with the filtrate sides facing the filter support (16) and the unfiltrate sides facing away from the filter support (16). Unfiltrate flow paths lead from the unfiltrate inlet of the top cover (12) to the unfiltrate side of the upper filter (40) and to the unfiltrate side of the lower filter (42), wherein blocking means prevent unfiltrate from flowing directly from the unfiltrate inlet of the top cover (12) to the filtrate outlet (22) of the bottom cover (14). Filtrate flow paths lead from the filtrate sides of the upper and lower filters (40, 42) to the filtrate outlet (22) of the bottom cover (14) via at least one filtrate guiding channel (48a, 48b) formed on the upper side of the filter support (16) and at least one filtrate guiding channel (48a, 48b) formed on the lower side of the filter support (16). The upper filter (40) and the lower filter (42) are identically shaped.

## Description

The invention relates to a filtration device, in particular a small-scale filtration device for production of biopharmaceuticals.

In the context of the present invention, a small-scale filtration device is to be understood as a device that is used for very small batches, ranging from some milliliters to a few liters, with an effective filtration area of about 5 to 100 cm². For example, an intravenous medication of such small volume may be filtered by a small-scale filtration device just before it is given to a patient. However, the concept of the invention may also be applied to larger filtration devices with an effective filtration area up to 300 cm² or even more.

From EP 2 363 197 A2 a filtration cartridge having a fluid inlet and a fluid outlet is known, which comprises a top end cap, a bottom end cap and a vent on at least one of the end caps. One or more filtration units are provided, each comprising two filtration plates. Each filtration plate has a membrane support plate having at least one filtration membrane respectively bonded to a bottom surface of the membrane support plate and to a top surface of the membrane support plate. The membrane support plates of the filtration plates are bonded to each other at an outer peripheral surface to form seals to prevent fluid feed from entering the fluid outlet except by having passed through at least one of the membranes. Fluid flow paths are provided from the fluid inlet, through the membranes and through the fluid outlet that prevent fluid feed from the inlet from bypassing the membranes and which provide a single permeate pathway including a row of holes for permeate to flow to the fluid outlet. The filtration membranes bonded to the bottom and top surfaces of the membrane support plate have different sizes and must not be confused with one another, thus making the assembly of the filtration cartridge complicated and prone to error.

It is an object of the invention to provide a small-scale filtration device, in particular for production of biopharmaceuticals, which is easy to assemble and provides a larger effective filtration area per membrane.

The above problem is solved by a filtration device according to claim 1 and a filter support for use in a filtration device according to claim 17. Advantageous and expedient embodiments of the invention are apparent from the respective dependent claims.

The invention provides a filtration device, in particular a small-scale filtration device for production of biopharmaceuticals, comprising a housing including a top cover having an unfiltrate inlet and a bottom cover having a filtrate outlet, and one or more filter supports stacked between the top cover and the bottom cover along a vertical direction such that an upper side of each filter support faces the top cover and a lower side of each filter support faces the bottom cover. Each filter support is equipped with a flat upper filter and a flat lower filter (such as membranes). Each filter has a filtrate side and an opposite unfiltrate side. The upper and lower filters are sealed to the upper and lower sides of the filter support, respectively, with the filtrate sides facing the filter support and the unfiltrate sides facing away from the filter support. Unfiltrate flow paths lead from the unfiltrate inlet of the top cover to the unfiltrate side of the upper filter and to the unfiltrate side of the lower filter, wherein blocking means prevent unfiltrate from flowing directly from the unfiltrate inlet of the top cover to the filtrate outlet of the bottom cover. Filtrate flow paths lead from the filtrate sides of the upper and lower filters to the filtrate outlet of the bottom cover via at least one filtrate guiding channel formed on the upper side of the filter support and at least one filtrate guiding channel formed on the lower side of the filter support. The upper filter and the lower filter are identically shaped.

In the assembled state the filtration device is one structural unit, i. e. the top cover, the bottom cover and the filter support(s) with the filters sealed thereon are connected, preferably welded together. Alternative connecting techniques include clamping, gluing, bonding, overmolding, mechanical assembly methods, such as screwing, optionally with sealing. In any event, the housing is a fluid-tight enclosure which can include a separate part or portion surrounding at least the filter supports, or the housing is formed by the top and bottom covers and outer portions of the filter supports, as will be explained further below.

With respect to the term "vertical direction", it is to be understood that the filtration device is designed such that gravity at least assists the flow of fluid to be filtered through the device from the unfiltrate inlet to the filtrate outlet. Accordingly, the use position of the filtration device is an upright position with the top cover located above the filter support(s) and the bottom cover. Of course, it is not necessary that the "vertical direction" is perfectly vertical.

The filters used in the filtration device are preferably flat filter membranes. However, depending on the application, other filter types, such as non-woven or woven filters could also be employed. The filters can be made of single layer filter materials having a thickness of approximately 100 µm, but it is also possible to use several layers of filter material or voluminous materials such as fiber materials, e. g. PP/PET meltblowns, spun bonds or short fiber wet-laids, having a thickness of up to 1000 µm, i. e. 10 times the thickness of a single layer filter material.

In the context of the invention, "sealed" preferably means that a tight connection is created that prevents especially bacteria and/or viruses from passing through. "Sealed" is not to be understood in a narrow sense and also includes connections made by techniques like welding, bonding, clamping, gluing, overmolding etc.

A key aspect of the invention is the use of identically shaped filters. This means that at least the upper and the lower filters sealed on the same filter support are identically shaped. Preferably all filters of the filtration device are identically shaped so that all filters to be used in the device can have the same geometric design. In this context "identically shaped" relates to the geometric shape, especially in the plane perpendicular to the vertical direction. This means that certain filter characteristics such as material, pore size, thickness in vertical direction etc., may differ as long as the horizontal dimensions and shapes, especially the contours, of the filters are identical. In other words: When viewed from the top or the bottom, the filters should be congruent.

The invention is based on the finding that assembly of a filtration device can be significantly simplified if identically shaped filters are used. With identically shaped filters there is no risk that a filter is applied to a wrong side of the filter support since the filter fits to both sides.

The filtrate guiding channels can extend in substantially horizontal directions and open into a drain hole of the filter support which vertically extends through the whole filter support and is fluidically connected with the filtrate outlet of the bottom cover. Of course, the filtrate guiding channels can be slightly inclined towards the drain hole to ensure effective draining of the filtrate.

According to a preferred embodiment of the filtration device, an upper side structure and a lower side structure are formed on the upper side and lower side of each filter support, respectively, both the upper side structure and the lower side structure forming an inner, preferably central, structure surrounding the perimeter of the drain hole. Each inner structure includes an inner welding area which is welded in a fluid-tight manner to a corresponding inner welding area of a neighboring filter support or to a corresponding inner welding area of the top cover or to a corresponding inner welding area of the bottom cover. The inner welding areas allow for a mechanical connection of the top and bottom covers and the filter supports stacked in-between at an inner region of the filtration device, which significantly contributes to the overall stability of the filtration device.

In general, the term "welding area" is not to be understood in a limiting sense. As already explained above, the top cover, the bottom cover, the filter support(s) and/or an annular wall of a housing of the filtration device may be connected to each other, preferably by welding, which is why the term "welding area" is used herein. However, the welding areas can be connected to each other by other connecting techniques, as mentioned above. This applies to both to the inner welding areas and the outer welding areas (which will be explained further below).

In order to allow a filter to be sealed at a location directly adjoining the inner welding area, the inner structure further includes an inner sealing area surrounding and adjoining the inner welding area. The inner sealing area is sealed to an inner area of the respective upper or lower filter. To ensure that a fluid space is established between neighboring filters of neighboring filter supports that can be reached by the unfiltrate, and to ensure that the inner sealing area does not interfere with the inner welding area, the inner sealing area is vertically offset relative to the inner welding area.

According to an advantageous design of the filtration device, both the inner welding area and the inner sealing area have a plurality of substantially identically shaped bulges and substantially identically shaped intermediate sections arranged alternately around the perimeter of the drain hole, the bulges extending in a horizontal direction from the drain hole. Depending on the number and concrete shape of the bulges and intermediate sections, the inner structure may look like a clover leaf, a triangle, a cross or a star, for example. With such a design it is possible that the fluid, after being filtered by a filter, can be guided to specific areas at the intermediate sections from where it can reach the drain hole.

Preferably, the numbers of the bulges and intermediate sections of the upper and lower side structures are the same, and the shapes and dimensions of the bulges and intermediate sections of the inner structures are substantially identical. This means that the overall shapes of the inner structures of the upper and lower sides of the filter support are basically identical. In view of the size of the filtration device, the total number of bulges on each side, which equals the total number of intermediate sections, should not be higher than 7, the preferred number being 3, 4 or 5.

In a further development of this preferred design, the inner structure of the upper side structure is arranged such that it exhibits a rotational offset around the vertical direction relative to the inner structure of the lower side structure, so that the bulges of the upper side structure are located opposite, preferably exactly opposite, the intermediate sections of the lower side structure and vice versa.

In the preferred design the filtrate guiding channels of the upper and lower sides of the filter support are circumferentially spaced from each other and lead, preferably alternatingly, to the bulges and to the intermediate sections of the upper and lower side structures. Most effective is a regular arrangement of the filtrate guiding channels at equal angular distances.

Preferably, the number of filtrate guiding channels equals the total number of bulges and intermediate sections of the inner structure, although this is not absolutely necessary.

It is advantageous if the fluid, once it has passed through a filter on one side of the filter support, can flow to the other side of the filter support, so that the filtrate, if necessary, can be guided to the drain hole on the other side of the filter support. For this purpose, at least one, preferably each, filtrate guiding channel of the upper side structure is located opposite a filtrate guiding channel of the lower side structure, and the opposite filtrate guiding channels are interconnected by a plurality of through openings allowing filtrate to pass from one filtrate guiding channel to the opposite filtrate guiding channel.

Given the opportunity that fluid can pass to an opposite filtrate guiding channel on the other side of the filter support, it is possible that on each side of the filter support only the filtrate guiding channels leading to the intermediate sections are fluidically connected with the drain hole. In other words: Fluid in a filtrate guiding channel that leads to a bulge and is not fluidically connected with the drain hole can still "switch sides" and flow to the drain hole via the respective opposite filtrate guiding channel which leads to an indentation. The fluidic connection of such a filtrate guiding channel with the drain hole can be established in that the filtrate guiding channel enters into the material of the filter support in front of the inner sealing area at the intermediate section on the one side and opens at the respective bulge on the opposite side of the filter support into an open space adjoining the drain hole.

To ensure a proper and stable (immovable) rest on the filter support, the upper and lower filters are sealed to outer sealing areas which are formed on the upper and lower sides of the filter support, respectively, and surround the filtrate guiding channels.

According to a first housing concept, the housing of the filtration device is formed, at least in part, by outer portions of the filter supports. In particular, the housing includes continuous outer welding areas formed on the upper and lower sides of each filter support. The outer welding area surrounds the outer sealing area and is welded in a fluid-tight manner to a corresponding outer welding area of a neighboring filter support or to a corresponding outer welding area of the top cover or to a corresponding outer welding area of the bottom cover. With this concept, an additional housing component is not necessary, and the overall size of the filtration device is minimized, only depending on the number of filter supports.

According to a second housing concept, the housing includes an annular wall surrounding at least the filter supports. This means that an additional housing component is provided which can ensure a great overall stability of the filtration device.

The filtration performance of the filtration device according to the invention can be further increased with an additional filter held by the bottom cover. In particular, the bottom cover is equipped with a flat bottom filter having a filtrate side and an opposite unfiltrate side. The bottom filter is sealed to an upper side of the bottom cover with the filtrate side facing the bottom cover and the unfiltrate side facing away from the bottom cover. A filtrate flow path leading from the filtrate side of the bottom filter to the filtrate outlet of the bottom cover, preferably via at least one filtrate guiding channel formed on the upper side of the bottom cover.

When the filtration area of the bottom filter on the bottom support is sufficient for the desired purpose of the filtration device, it is possible to do without any filter support. In this case, the filtration device does not comprise any filter support, and the top cover is directly welded to the bottom cover.

Contrary to known devices and irrespective of the actual number of filter supports, the filtrate flow path leading from the filtrate side of the bottom filter to the filtrate outlet of the bottom cover ensures that the hold-up volume at the bottom of the filtration device is minimized. This is especially important in case of expensive drugs.

The invention also provides a filter support for use in a filtration device. The filter support is adapted to be equipped with a flat upper filter and a flat lower filter, the upper and lower filters being shaped identically. A filtrate flow path leads from the filtrate sides of the upper and lower filters to the filtrate outlet of the bottom cover via at least one filtrate guiding channel formed on the upper side of the filter support and at least one filtrate guiding channel formed on the lower side of the filter support. The other features of the filter support are preferably exactly as described above in connection with the entire filtration device.

In general, the invention achieves a significant reduction of the hold-up volume (dead volume) and a more efficient utilization of the filter area. It is also to be noted that the lower filter on a filter support is positioned such that upon application of air pressure the drug (or whatever medium is to be filtered) can be fully urged through the filter, thereby achieving the maximum yield.

Further features and advantages of the invention will become apparent from the following description and from the accompanying drawings to which reference is made. In the drawings:
- Figure 1 shows an exploded view of components of a small-scale filtration device according to a first embodiment of the invention;
- Figure 2 shows a perspective top view of the assembled filtration device;
- Figure 3 shows a perspective bottom view of the assembled filtration device;
- Figure 4 shows a cross-section side view of the assembled filtration device;
- Figure 5 shows a perspective view of one side of a filter support of the filtration device;
- Figure 6 shows a perspective view of the opposite side of the filter support of Figure 5;
- Figure 7 shows a detailed top view of one side of a filter support of the filtration device;
- Figure 8 shows an illustration of fluid flowing along a primary filtrate guiding channel in a detailed top view of the filter support;
- Figure 9 shows a cross-section view along line A-A in Figure 8 with filters on both sides of the filter support;
- Figure 10 shows an illustration of fluid flowing along a secondary filtrate guiding channel in a detailed top view of the filter support;
- Figure 11 shows a cross-section view along line B-B in Figure 10 with filters on both sides of the filter support;
- Figure 12 shows an illustration of fluid flow in a detailed cross-section view of a filtration device with three stacked filter supports;
- Figure 13 shows an illustration of fluid flowing along a filtrate guiding channel in a detailed top view of the bottom cover of the filtration device;
- Figure 14 shows a cross-section view along line C-C in Figure 13 with a filter on the upper side of the bottom cover;
- Figure 15 shows a cutaway side view of a filtration device without any filter support;
- Figure 16 shows a perspective cutaway side view of a filtration device with nine filter supports accommodated in a separate housing;
- Figure 17 shows a perspective top view of an assembled small-scale filtration device according to a second embodiment of the invention;
- Figure 18 shows an exploded top view of components of the filtration device of Figure 17;
- Figure 19 shows an exploded bottom view of components of the filtration device;
- Figure 20 shows a top view of the uppermost filter support of the filtration device of Figure 17 with a filter on the upper side of the filter support;
- Figure 21 shows a perspective cross-section side view of the filtration device; and
- Figures 22A, 22B and 22C show three illustrations of possible fluid paths through the filtration device in different exploded views without filters.

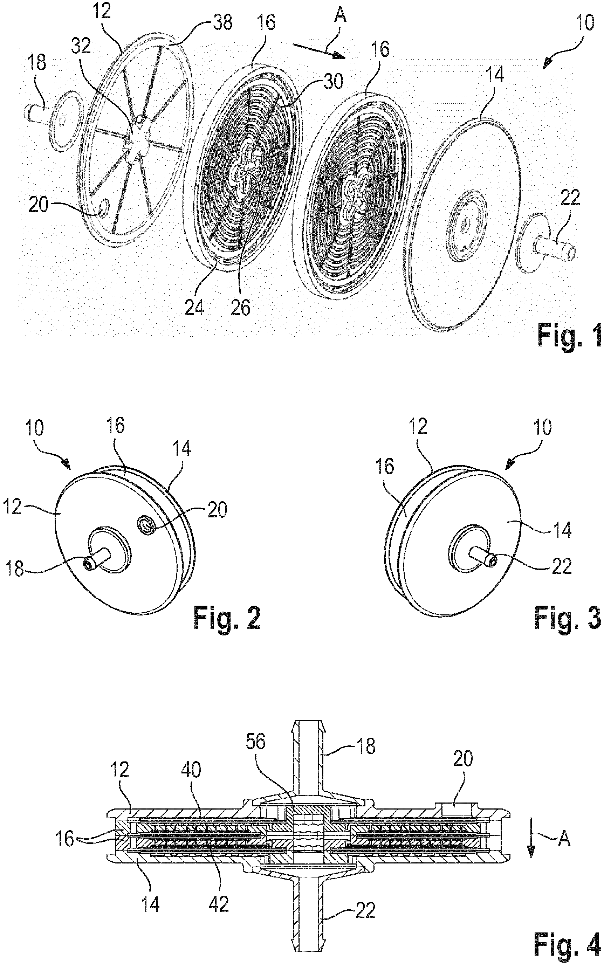

Figures 1 to 4 depict a first embodiment of a small-scale filtration device 10 comprising a top cover 12, a bottom cover 14 and a number of identically shaped filter supports 16 stacked between the top cover 12 and the bottom cover 14. The actual number of filter supports 16 depends on the desired total effective filtration area and may be only one or even zero, as will be explained later. The arrangement of the bottom cover 14, the stacking of the filter supports 16 (if any), and the top cover 14 define a vertical direction A.

The top cover 12 includes an unfiltrate inlet 18, here in the form of an inlet connector, and an optional vent opening 20. The bottom cover 14 includes a filtrate outlet 22, here in the form of an outlet connector. The unfiltrate inlet 18 and the filtrate outlet 22 may be formed in one piece with the top cover 12 and the bottom cover 14, respectively, or they are attached later. Likewise, a vent pipe may be formed with or attached to the vent opening 20 of the top cover 12.

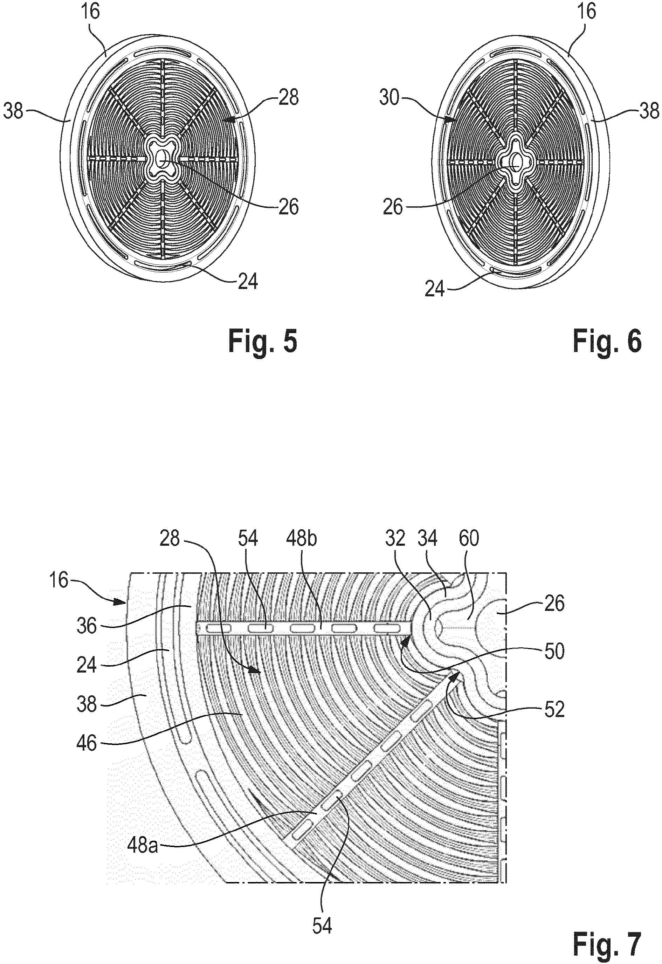

A single filter support 16 is shown in Figures 5 and 6. The filter support 16 is generally flat and disc-shaped, but other shapes are also possible. Several distribution holes 24 are formed in the periphery of the filter support 16, each extending vertically through the whole filter support 16 and being fluidically connected with the unfiltrate inlet 18 of the top cover 12. A (preferably central) drain hole 26 also extends vertically through the whole filter support 16. The drain hole 26 is fluidically connected with the filtrate outlet 22 of the bottom cover 14.

In the assembled state, an upper side of the filter support 16 faces the top cover 12 and a lower side of the filter support 16 faces the bottom cover 14. On the upper and lower sides of the filter support 16 an upper side structure 28 and a lower side structure 30 are formed, respectively. Further details of the identically shaped upper and lower side structures 28, 30 become apparent from Figure 7.

Both the upper side structure 28 and the lower side structure 30 form an inner structure surrounding the perimeter of the drain hole 26. The inner structure includes a continuous circumferential inner welding area 32. Depending on the position of the filter support 16 in the filtration device 10, the upward facing inner welding area 32 of the upper side structure 28 is welded in a fluid-tight manner to a corresponding downward facing and likewise oriented inner welding area 32 either of the top cover 12 or of a neighboring filter support 16 above, while the downward facing inner welding area 32 of the lower side structure 30 is welded in a fluid-tight manner to a corresponding upward facing and likewise oriented inner welding area 32 either of the bottom cover 14 or of a neighboring filter support 16 below. Ideally, the inner welding areas 32 that are welded together are congruent.

The inner structure further includes a continuous circumferential inner sealing area 34 which preferably directly adjoins and surrounds the inner welding area 32. Relative to the inner welding area 32, the inner sealing area 34 is vertically offset. In particular, with respect to the orientation in the assembled state of the filtration device 10, the inner welding area 32 of the upper side structure 28 is at a lower vertical level than the adjoining inner sealing area 34, while the inner welding area 32 of the lower side structure 30 is at a higher vertical level than the adjoining inner sealing area 34. The difference in vertical height between the inner welding area 32 and the inner sealing area 34 on both sides of the filter support 16 is adapted to the thickness of the filters to be used, as will be explained later.

The upper and lower side structures 28, 30 form outer structures on the upper and lower sides at the periphery of the filter support 16, including outer sealing areas 36 and continuous circumferential outer welding areas 38 surrounding the outer sealing areas 36. Depending on the position of the filter support 16 in the filtration device 10, the upward facing outer welding area 38 of the upper side structure 28 is welded in a fluid-tight manner either to a downward facing corresponding outer welding area 38 of the top cover 12 or to a downward facing corresponding outer welding area 38 of a neighboring filter support 16 above, while the downward facing outer welding area 38 of the lower side structure 30 is welded in a fluid-tight manner either to an upward facing corresponding outer welding area 38 of the bottom cover 14 or to an upward facing corresponding outer welding area 38 of a neighboring filter support 16 below. Ideally, the outer welding areas 38 that are welded together are congruent. Thus, the top and bottom covers 12, 14 together with the connected outer welding areas 38 of the filter supports 16 form a housing of the filtration device 10.

The inner and outer sealing areas 34, 36 of the filter support 16 are sealed to flat filters 40, 42, especially filter membranes (see Figures 4, 9 and 11). In particular, the inner sealing area 34 on the upper side of the filter support 16 is sealed to an inner area of a filtrate side of an upper filter 40, while the outer sealing area 36 on the upper side of the filter support 16 is sealed to an outer area of the filtrate side of the upper filter 40. Likewise, the inner sealing area 34 on the lower side of the filter support 16 is sealed to an inner area of a filtrate side of an identically shaped lower filter 42, while the outer sealing area 36 on the lower side of the filter support 16 is sealed to an outer area of the filtrate side of the lower filter 42. This means that each filter support 16 is equipped with two identically shaped filters 40, 42, one on each side of the filter support 16, with the unfiltrate sides of both filters facing away from the filter support 16.

As already mentioned, the filters 40, 42 can be a single layer filter material, or they can include multiple layers of filter material. The geometry of the filter support 16, especially the vertical offsets between the inner welding areas 32 and the inner sealing areas 34, are adapted to the thickness of the filters on both sides accordingly.

Between the inner and outer sealing areas 34, 36 the filters 40, 42 cover a plurality of filtrate collecting channels 46, which lead to filtrate guiding channels 48a, 48b, both formed by the respective upper or lower side structure 28, 30 of the filter support 16. The filtrate collecting channels 46 extend in substantially horizontal directions, but preferably they are slightly inclined towards the filtrate guiding channels 48a, 48b. Filtrate collecting channels 46 may be formed by substantially parallel webs. Such webs can be formed as continuous walls (thereby providing solid support to the sealed filter 40 or 42) and/or as interrupted walls (thereby reducing the contact area of the filter 40 or 42 with the filtrate collecting channels 46, resulting in an increased effective filter area).

As can be seen in Figures 5 to 7, on both sides of the filter support 16 the inner structure, i. e. both the inner welding area 32 and the inner sealing area 34, has a plurality of substantially identically shaped bulges 50 and substantially identically shaped intermediate sections 52 arranged alternately around the perimeter of the drain hole 26. While the bulges 50 extend in radial horizontal directions away from the drain hole 26, the intermediate sections 52 are formed as indentations extending in radial horizontal directions towards the drain hole 26.

In the embodiment shown here, the number of bulges 50 and intermediate sections 52 on each side of the filter support 16 is 4, respectively, thus forming a shape similar to a four-leaf clover. However, the number of regularly arranged bulges 50 and intermediate sections 52 can be 3 or more than 4, but preferably not more than 7. Moreover, the intermediate sections 52 may also be formed as straight sections without any indentations. The bulges 50 and indentations (if any) can have a rounded contour, or they can have sharp corners, such as in a triangle or square, for example.

In any event, the bulges 50 and intermediate sections 52 of the inner structure form a shape having rotational symmetry with respect to the vertical direction A. The shape's degree of rotational symmetry corresponds to the number of bulges 50. In the case of the four-leaf clover shape the degree of rotational symmetry is 4, because after each 90° rotation the shape looks exactly the same.

In the embodiment shown here the number of filtrate guiding channels 48a, 48b corresponds to the total number of bulges 50 and intermediate sections 52. The filtrate guiding channels 48a, 48b are circumferentially spaced from each other, each leading in a substantially radial direction from the outer sealing area 36 to either a bulge 50 or an intermediate section 52 of the inner structure. However, it is not necessary that all of the bulges 50 or intermediate sections 52 are associated with a filtrate guiding channel 48a or 48b.

Only the filtrate guiding channels 48a leading to intermediate sections 52, hereinafter referred to as primary filtrate guiding channels 48a, are directly fluidically connected with the drain hole 26. The filtrate guiding channels 48b leading to bulges 50, hereinafter referred to as secondary filtrate guiding channels 48b, terminate at the elevated inner sealing area 34 of the inner structure and are not directly fluidically connected with the drain hole 26. Details of the course of the primary and secondary filtrate guiding channels 48a, 48b at the inner area will be explained later in connection with the description of the operation of the filtration device 10.

As already mentioned, the upper and lower side structures 28, 30 of the filter support 16 are substantially identical, at least with respect to the inner structure and at least some of the filtrate guiding channels 48a, 48b. However, the upper and lower side structures 28, 30 are arranged with a rotational offset relative to each other. In particular, the inner structures of the upper and lower side structures 28, 30 are arranged such that the bulges 50 of the upper side structure 28 are located opposite the intermediate sections 52 of the lower side structure 30, and the intermediate sections 52 of the upper side structure 28 are located opposite the bulges 50 of the lower side structure 30.

Due to this rotational offset between the upper and lower side structures 28, 30, especially with respect to the inner sealing areas 34, the filter supports 16 are stacked on top of each other with the same rotational offset, because the inner sealing areas 34 of opposing side structures 28, 30 need to match each other; ideally they are congruent when they are welded together.

Primary filtrate guiding channels 48a on one side of the filter support 16 are located exactly opposite secondary filtrate guiding channels 48b on the other side of the filter support 16. Several through openings 54 are formed in the filtrate guiding channels 48a, 48b so that fluid can travel from a secondary filtrate guiding channel 48b on one side to a primary filtrate guiding channel 48a on the other side (and vice versa, although this is not intended as will become apparent from the description of the operation of the filtrations device further below).

The upper side of the bottom cover 14 has an upper side structure 28 similar to the upper side structures 28 of the filter supports 16, including an inner structure with an inner welding area 32 and an inner sealing area 34, and an outer structure including an outer welding area 38 and an outer sealing area 36. The upper side structure 28 also includes filtrate collecting channels 46 and primary filtrate guiding channels 48a, but not necessarily secondary filtrate guiding channels 48b. A flat bottom filter 44, which is preferably shaped exactly like the upper and lower filters 40, 42 on the filter supports 16, is sealed to the inner and outer sealing areas 34, 36 of the bottom cover 14 so that the unfiltrate side of the bottom filter 44 faces upwards, i. e. away from the bottom cover 14.

It is to be noted that the similar, but alternating rotationally offset upper and lower side structures 28, 30 of the filter supports 16 and the upper side structure 28 of the bottom cover 14 allow the use of identically shaped filters 40, 42, 44. In particular, all filters 40, 42, 44 have the same two-dimensional shape, including an inner contour adapted to the contour of the inner sealing areas 34. With respect to the inner contour, the alternating rotational orientations of the filters 40, 42, 44 are automatically given by the rotational orientation of the respective contour of the inner sealing area 34 to which the respective filter 40, 42, 44 is sealed, while it is not necessary to distinguish between upper and lower and bottom filters 40, 42, 44 during assembly of the filtration device 10.

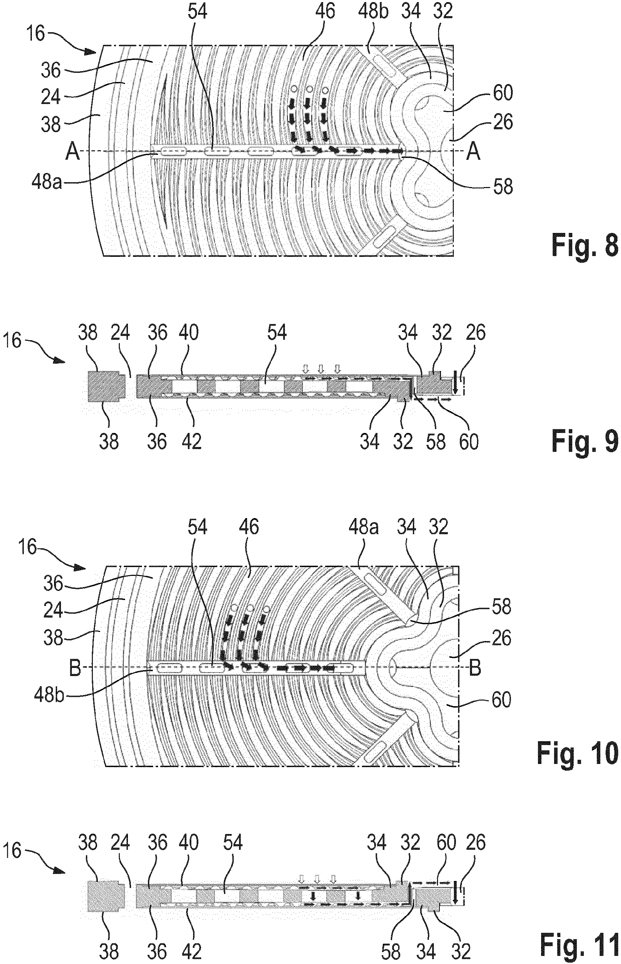

In the following, the flow of fluid through the filtration device 10, which is caused by gravity and system pressure, is explained with additional reference to Figures 8 to 14.

The filtration device 10 is held such that the top cover 12 is located above the bottom cover 14 and the direction A is oriented substantially vertically. The fluid to be filtered (unfiltrate) is supplied to the unfiltrate inlet 18 of the top cover 12. Along unfiltrate flow paths the unfiltrate flows to the unfiltrate sides of the filters 40, 42, 44. Blocking means 56, such as the roof-like shaped structure shown in Figure 4, prevent unfiltrate from flowing directly from the unfiltrate inlet 18 through the drain holes 26 of the filter supports 16 to the filtrate outlet 22 of the bottom cover 14. The distribution holes 24 in the filter supports 16 ensure that the unfiltrate is distributed to the unfiltrate sides of all filters 40, 42, 44, including the bottom filter 44 sealed to the upper side of the bottom cover 14.

The vertical offset between the inner sealing area 34 and the inner welding area 32 is, on the one hand, large enough to allow the unfiltrate to enter a space between a lower filter 42 on the lower side of a filter support 16 and an upper filter 40 on an upper side of an adjoining filter support 16 located below; on the other hand, this space is low enough to allow a very compact design.

When the fluid passes through the filters 40, 42 sealed to the filter supports 16, it is filtered, thus becoming filtrate. The fluid can pass through an upper filter 40 in a downward direction, and/or - supported by the system pressure - through a lower filter 42 in an upward direction. The filtrate is collected by the filtrate collecting channels 46 of the filter supports 16 and flows into the filtrate guiding channels 48a, 48b.

By way of example, Figures 8 to 11 show the path of fluid passing through a filter 40 or 42 and being received by filtrate collecting channels 46 of a filter support 16, White arrows indicate the flow direction of the unfiltrate before passing the filter 40 or 42, and black arrows indicate the flow direction of filtrate after having passed the filter 40 or 42. For sake of simplicity, three locations where fluid has passed through an upper filter 40 are shown as small white circles in Figures 8 and 10. Gravity, the inclination of the filtrate collecting channels 46 and system pressure cause the filtrate received in the filtrate collecting channels 46 to flow into the filtrate guiding channels 48a, 48b.

Figures 8 and 9 show the flow of filtrate in a primary filtrate guiding channel 48a leading to an intermediate section 52 of the inner structure. As can be seen in Figure 9, the primary filtrate guiding channel 48a enters into a passage 58 in the material of the filter support 16 in front of the inner sealing area 34 which opens at the respective bulge 50 on the opposite side of the filter support 16 into an open space 60 adjoining the drain hole 26. Thus, the filtrate can directly flow to the filtrate outlet 22 via the drain hole 26 of the filter support 16 (and via the drain holes 26 of further filter supports 16 located below, if any).

Figures 10 and 11 show the flow of filtrate in a secondary filtrate guiding channel 48b leading to a bulge 50 of the inner structure. As can be seen in Figure 11, the secondary filtrate guiding channel 48b is not in direct fluid communication with the drain hole 26 as it ends at a vertical wall delimiting the inner sealing area 34. However, the filtrate can pass through the through openings 54 into the opposite primary filtrate guiding channel 48a. From there the filtrate can flow into the drain hole 26 as described above.

According to an alternative variant, which is not shown in the Figures, filtrate could also pass to the other side of the filter support 16 via one or more interruptions in the web-like inner welding area 32. This means that the filtrate passes through the inner welding area 32 and then flows to the other side of the filter support 16. Of course, such a flow path requires that the inner sealing area 32 remains fluid-tight with respect to its surroundings.

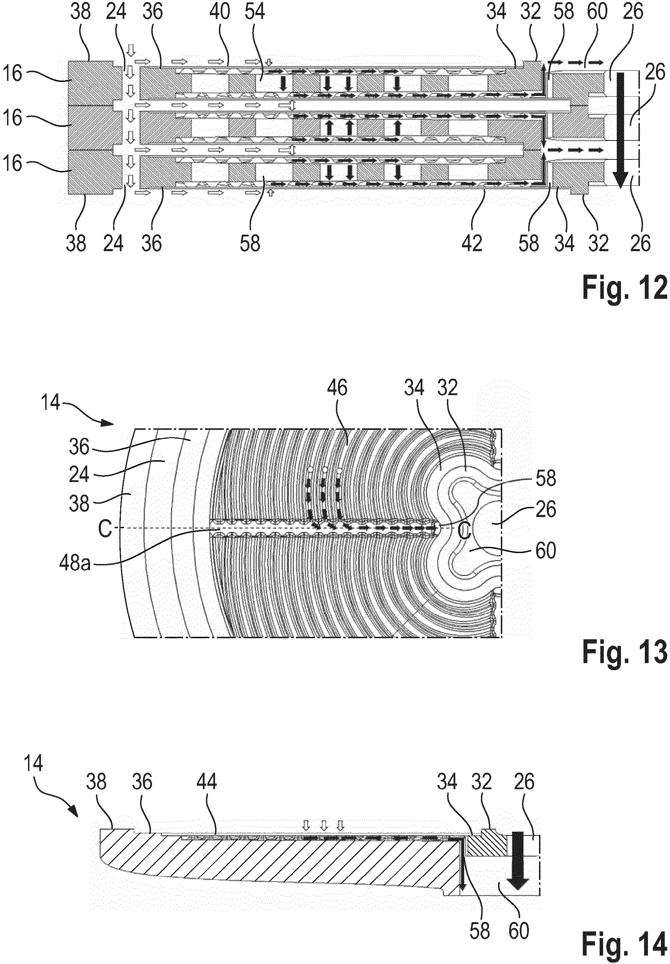

For the purpose of illustrating the general flow concept of the filter supports 16, Figure 12 shows exemplary flow paths with three filter supports 16 stacked on each other.

Figures 13 and 14 illustrates the flow path of fluid passing through the bottom filter 44 on the bottom cover 14. As mentioned above, the upper side structure 28 of the bottom cover 14 does not include any secondary filtrate guiding channels 48b, as it is not intended that the filtrate switches to a lower side structure 30. Accordingly, the flow path substantially corresponds to the one shown in Figures 8 and 9. Since the primary filtrate guiding channels 48a of the upper side structure 28 of the bottom cover 14 are in direct fluid communication with the filtrate outlet 22, there is almost no hold-up volume below the bottom filter 44. In fact, due to its design, the filtration device 10 is automatically almost completely drained after use.

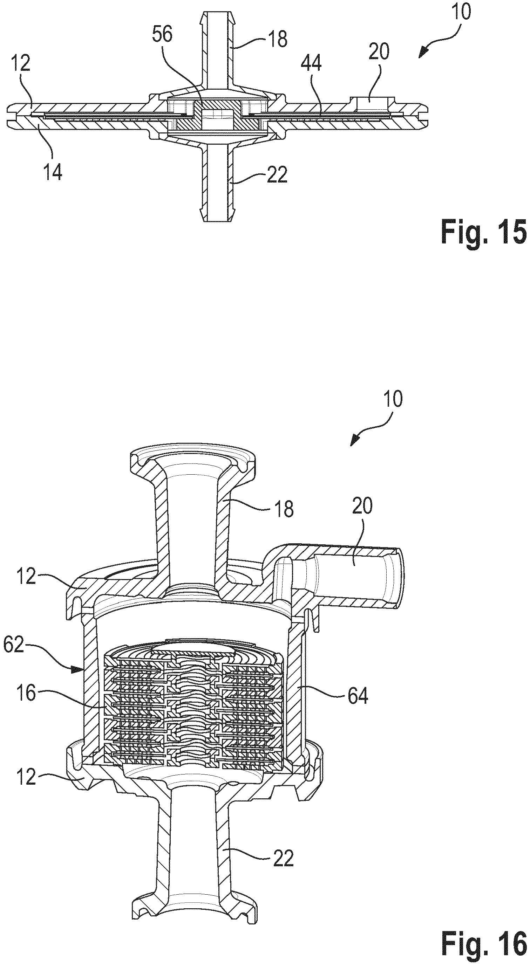

Figure 15 shows a simplified variant of the first embodiment without any filter supports 16. The top cover 12 is directly connected to the bottom cover 14 via their inner and outer welding areas 32, 38. The only filter of this variant is the bottom filter 44 sealed to the inner and outer sealing areas 34, 36 of the upper side structure 28 of the bottom cover 14. The unfiltrate supplied to the unfiltrate inlet 18 flows, via unfiltrate flow paths, directly to the unfiltrate side of the bottom filter 44. The further flow path through the bottom filter 44 to the filtrate outlet 22 is as described above with reference to Figures 13 and 14.

The embodiments shown in Figures 1 to 14 and in Figure 15 are particularly advantageous compared to known filtration device since the hold-up volume is minimized. This is especially important when only a small amount of medium is available and/or when the medium is of high monetary value.

Another variant of the first embodiment is shown in Figure 16. The filtration device 10 comprises a separate housing 62 with an annular wall 64 that surrounds at least the filter supports 16. The outer welding areas 38 of the top and bottom covers 12, 14 are welded in a fluid-tight manner to axial end faces of the annular wall 64. Preferably, but not necessarily, the outer welding areas 38 of the top and bottom covers 12, 14 are congruent with the outer welding areas 38 of the filter supports 16.

In the embodiment shown in Figure 16, the filter supports 16 preferably do not have any outer welding areas 38 at all. Outer welding areas 38 are not needed here to form a housing, since the annular wall 64 takes over this task.

Furthermore, the bottom cover 14 does not need to have any filtrate collecting channels 46 and filtrate guiding channels 48 on its upper side, even though this would theoretically be possible. As shown in Figure 16, the filter supports 16 are inserted into an existing housing 62 with a bottom cover 14 which has no special configuration. Instead, the upper side of the lowermost filter support 16 takes over this task. A filter 40 is sealed here only on the upper side of the lowermost filter support 16. The lower side of this filter support 16 is welded to the bottom cover 14 in such a way that a fluid-tight barrier is created and no unfiltrate can reach the filtrate collecting channels 46 and the filtrate guiding channels 48 on the lower side. Alternatively, at least the lowermost filter support 16 could be welded to the annular wall 64 instead of the bottom cover 14.

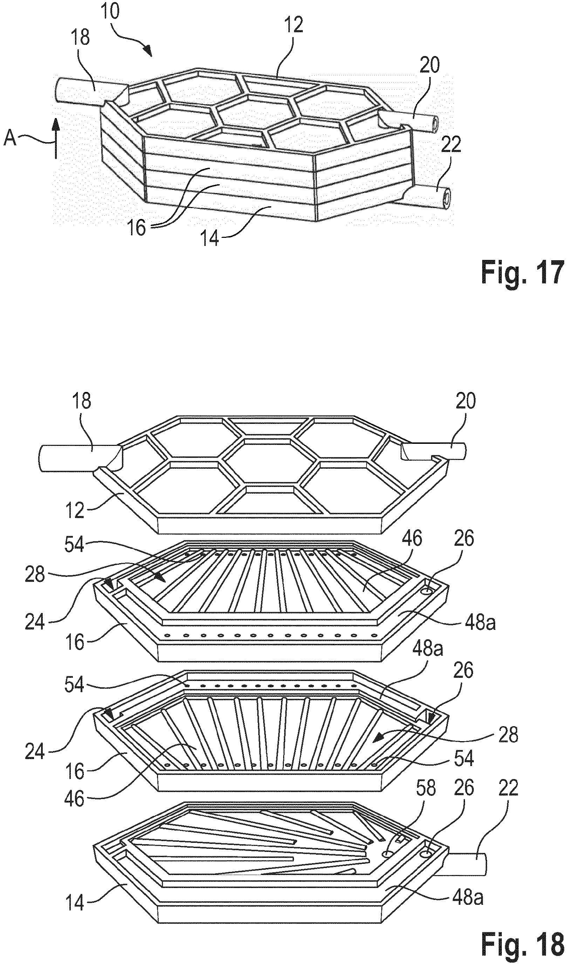

Figures 17 to 22 show a second embodiment of a small-scale filtration device 10. Components and parts of the filtration device 10 having the same functions as in the first embodiment are given the same reference signs and are not explained again in detail.

In the second embodiment the top and bottom covers 12, 14 and the filter supports 16 are not generally circular as in the first embodiment. Rather, their outer contours substantially have the shape of honeycombs.

Contrary to the first embodiment, the drain hole 26 of each filter support 16, which is fluidically connected to the filtrate outlet 22, is not a central hole. Rather, the drain hole 26 is located at a vertex of the filter support 16. At the opposite vertex a distribution hole 24 is provided, which is fluidically connected with the unfiltrate inlet 18.

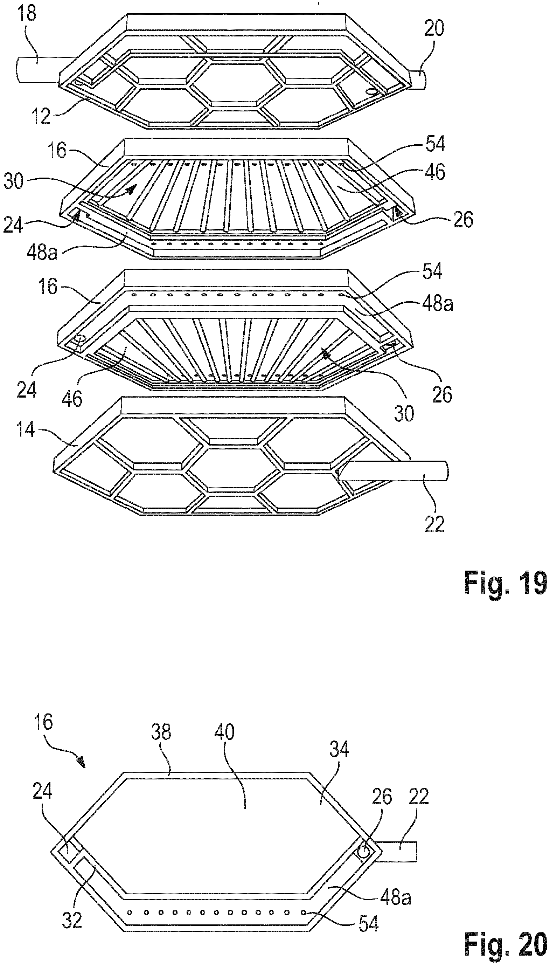

As can be seen in Figures 18 and 19, the generally flat filter supports 16 have upper and lower side structures 28, 30 which are substantially identical. In particular, a lower side structure 30 of a filter support 16 looks like an upper side structure 28 of the same filter support 16 that is rotated 180° about an axis extending through the drain hole 26 and the distribution hole 24.

The filtrate collecting channels 46 formed by the upper and lower side structures 28, 30 of the filter supports 16 are inclined and lead to through openings 54, which are located along a lateral side of the filter support 16 between the drain hole 26 and the distribution hole 24. The through openings 54 allow fluid to pass to a fluid guiding channel 48a on the respective other side of the filter support 16 which is fluidically separated from the filtrate collecting channels 46 by the elevated outer sealing area 36. The fluid guiding channels 48a on both sides of the filter support 16 are in fluid communication with the drain hole 26 of the filter support 16. Thus, the fluid guiding channels 48a functionally correspond to the primary fluid guiding channels 48a of the first embodiment.

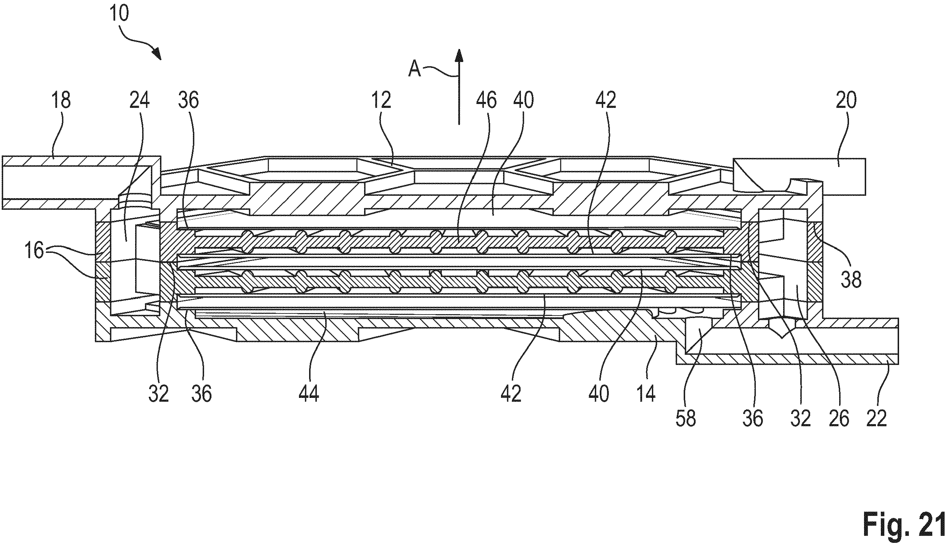

As can be seen in Figure 18, the upper side structure 28 of the bottom cover 14 is basically identical in shape and orientation with the upper side structure 28 of the next-but-one filter support 16 (here: the upper filter support 16), except for the filtrate collecting channels 46 and the through openings 54. In particular, the filtrate collecting channels 46 of the bottom cover 14 run in a different direction (in particular turned by 90°) compared to the filtrate collecting channels 46 of the filter supports 16. The filtrate collecting channels 46 of the bottom cover 14 are inclined and lead to a passage 58 which, in turn, leads to the filtrate outlet 22 (see Figure 21).

Figure 20 shows a filter support 16 with a flat upper filter 40 (membrane) sealed to the outer sealing area 36 of the upper side structure 28 surrounding the filtrate collecting channels 46 and the associated through openings 54. Accordingly, the filter 40 does not cover the filtrate guiding channel 48a. An identically shaped flat lower filter 32 is sealed in the same way to the outer sealing area 36 of the lower side structure 30.

Similar to the first embodiment, outer welding areas 38 are formed at the periphery of the filter support 16 on both sides thereof, as well as on the lower side of the top cover 12 and on the upper side of the bottom cover 14.

It is to be noted that in the second embodiment there are no inner sealing areas 34.

It is further to be noted that two consecutive filter supports 16 are actually not identical, but are mirrored to each other. Otherwise the distribution hole 24 and the drain hole 26 would change sides. However, the filters 40, 42 are always the same size and shape (identical) since they are rotationally symmetrical. The mirrored arrangement of consecutive filter supports 16 also means that the embodiment shown in Figures 17 - 22 can only be operated with an even number of filter supports 16, unless two different (mirrored) bottoms are available, in which case the number of filter supports 16 is flexible.

In Figure 21 a cross-section view of an assembled filtration device 10 according to the second embodiment with two filter supports 16 stacked upon each other is shown.

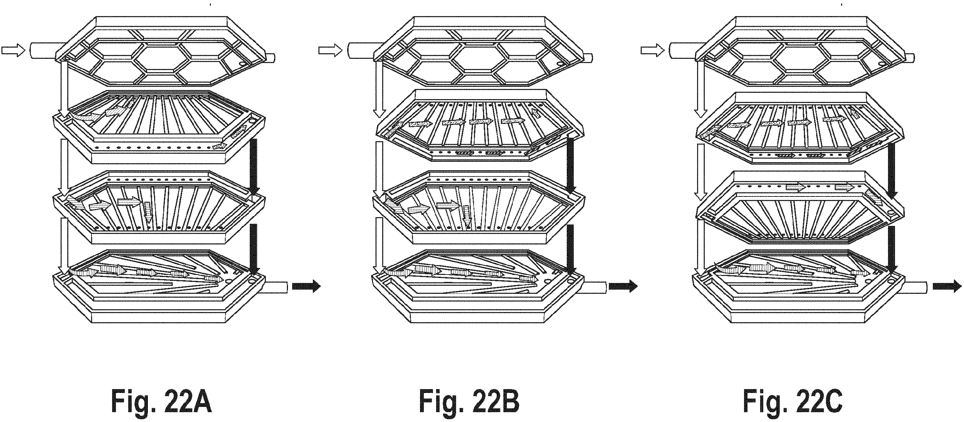

Figures 22A, 22B and 22C show an exemplary flow of fluid through the filtration device 10 of Figure 21, which is caused by gravity and system pressure. It is to be noted that Figures 22A, 22B and 22C only differ in the way that the stacked filter supports 16 are shown: In Figure 22A the upper sides of both filter supports 16 are visible. In Figure 22B the lower side of the upper filter support 16 and the upper side of the lower filter support 16 are visible. In Figure 22C the lower sides of both filter supports 16 are visible.

White arrows indicate unfiltrate coming from the unfiltrate inlet 18. Diagonally hashed arrows in Figure 22A indicate flow of unfiltrate passing over the upper filter 40 on the upper side of the upper filter support 16, before it passes through the filter 40 and becomes filtrate. The filtrate is collected by the filtrate collecting channels 46 on the upper side and flows to and through the associated through openings 54 into the filtrate guiding channel 48a on the lower side of the filter support 16. Via the filtrate guiding channel 48a and the drain holes 26 the filtrate flows to the filtrate outlet 22, as shown by the black arrows in Figure 22B or Figure 22C.

Dotted arrows in Figure in 22B or Figure 22C indicate unfiltrate passing over the lower filter 42 on the lower side of the upper filter support 16, before it passes through the filter 42 and becomes filtrate. The filtrate is collected by the filtrate collecting channels 46 on the lower side and flows to and through the associated through openings 54 into the filtrate guiding channel 48a on the upper side of the filter support 16. Via the filtrate guiding channel 48a and the drain holes 26 the filtrate flows to the filtrate outlet 22, as shown by the black arrows in Figure 22A.

Horizontally hashed arrows in Figure in 22A or Figure 22B indicate unfiltrate passing over the upper filter 40 on the upper side of the lower filter support 16, before it passes through the filter 40 and becomes filtrate. The filtrate is collected by the filtrate collecting channels 46 on the upper side and flows to and through the associated through openings 54 into the filtrate guiding channel 48a on the lower side of the filter support 16. Via the filtrate guiding channel 48a and the drain holes 26 the filtrate flows to the filtrate outlet 22, as shown by the black arrows in Figure 22C.

Vertically hashed arrows in Figure in 22A or Figure 22B or Figure 22C indicate unfiltrate passing over the bottom filter 44 on the upper side of the bottom cover 14, before it passes through the filter 44 and becomes filtrate. The filtrate is collected by the filtrate collecting channels 46 on the upper side and flows into the passage 58 that leads to the filtrate outlet 22 (see Figure 21).

The filtration device 10 is conceived as a single-use device. Accordingly, all components of the filtration device 10 are made from plastic materials which are suitable for typical sterilization techniques, especially gamma sterilization (irradiation) and chemical sterilization, e.g. with ethylene oxide (ETO). "Suitable" here means that the relevant material properties, such as stability, brittleness etc., are not significantly affected by the sterilization process.

The principles of the invention are not limited to the above-described embodiments of the filtration device 10 but can also be applied to other suitable designs.

### List of Reference Signs

- 10: filtration device
- 12: top cover
- 14: bottom cover
- 16: filter support
- 18: unfiltrate inlet
- 20: vent opening
- 22: filtrate outlet
- 24: distribution hole
- 26: drain hole
- 28: upper side structure
- 30: lower side structure
- 32: inner welding area
- 34: inner sealing area
- 36: outer sealing area
- 38: outer welding area
- 40: upper filter
- 42: lower filter
- 44: bottom filter
- 46: filtrate collecting channel
- 48a: primary filtrate guiding channel
- 48b: secondary filtrate guiding channel
- 50: bulge
- 52: intermediate section
- 54: through opening
- 56: blocking means
- 58: passage
- 60: open space
- 62: housing
- 64: annular wall

## Claims

1. A filtration device (10), in particular a small-scale filtration device for production of biopharmaceuticals, the filtration device (10) comprising
a housing including a top cover (12) having an unfiltrate inlet (18) and a bottom cover (14) having a filtrate outlet (22); and
one or more filter supports (16) stacked between the top cover (12) and the bottom cover (14) along a vertical direction (A) such that an upper side of each filter support (16) faces the top cover (12) and a lower side of each filter support (16) faces the bottom cover (14);
each filter support (16) being equipped with a flat upper filter (40) and a flat lower filter (42), each filter (40, 42 ) having a filtrate side and an opposite unfiltrate side;
the upper and lower filters (40, 42) being sealed to the upper and lower sides of the filter support (16), respectively, with the filtrate sides facing the filter support (16) and the unfiltrate sides facing away from the filter support (16);
unfiltrate flow paths leading from the unfiltrate inlet of the top cover (12) to the unfiltrate side of the upper filter (40) and to the unfiltrate side of the lower filter (42), wherein blocking means prevent unfiltrate from flowing directly from the unfiltrate inlet of the top cover (12) to the filtrate outlet (22) of the bottom cover (14);
filtrate flow paths leading from the filtrate sides of the upper and lower filters (40, 42) to the filtrate outlet (22) of the bottom cover (14) via at least one filtrate guiding channel (48a, 48b) formed on the upper side of the filter support (16) and at least one filtrate guiding channel (48a, 48b) formed on the lower side of the filter support (16);
**characterized in that** the upper filter (40) and the lower filter (42) are identically shaped.

2. The filtration device (10) according to claim 1, **characterized in that** the filtrate guiding channels (48a, 48b) extend in substantially horizontal directions and are fluidically connected to a drain hole (26) of the filter support (16) which vertically extends through the whole filter support (16) and is fluidically connected with the filtrate outlet (22) of the bottom cover (14).

3. The filtration device (10) according to claim 2, **characterized in that** an upper side structure (28) and a lower side structure (30) are formed on the upper side and lower side of each filter support (16), respectively, both the upper side structure (28) and the lower side structure (30) forming an inner structure surrounding the perimeter of the drain hole (26), each inner structure including an inner welding area (32) which is welded in a fluid-tight manner to a corresponding inner welding area (32) of a neighboring filter support (16) or to a corresponding inner welding area (32) of the top cover (12) or to a corresponding inner welding area (32) of the bottom cover (14).

4. The filtration device (10) according to claim 3, **characterized in that** the inner structure further includes an inner sealing area (34) which is sealed to an inner area of the upper filter (40) or the lower filter (42), the inner sealing area (34) surrounding and adjoining the inner welding area (32) and being vertically offset relative to the inner welding area (32).

5. The filtration device (10) according to claim 4, **characterized in that** both the inner welding area (32) and the inner sealing area (34) have a plurality of substantially identically shaped bulges (50) and substantially identically shaped intermediate sections (52) arranged alternately around the perimeter of the drain hole (26), the bulges (50) extending in a horizontal direction from the drain hole (26).

6. The filtration device (10) according to claim 5, **characterized in that** the numbers of the bulges (50) and intermediate sections (52) of the upper and lower side structures (28, 30) are the same.

7. The filtration device (10) according to claim 6, **characterized in that** the inner structure of the upper side structure (28) is arranged such that it exhibits a rotational offset around the vertical direction relative to the inner structure of the lower side structure (30), so that the bulges (50) of the upper side structure (28) are located opposite the intermediate sections (52) of the lower side structure (30) and vice versa.

8. The filtration device (10) according to claim 7, **characterized in that** the filtrate guiding channels (48a, 48b) of the upper and lower sides of the filter support (16) are circumferentially spaced from each other, the filtrate guiding channels (48a, 48b) leading, preferably alternatingly, to the bulges (50) and to the intermediate sections (52) of the upper and lower side structures (28, 30).

9. The filtration device (10) according to claim 8, **characterized in that** the number of filtrate guiding channels (48a, 48b) equals the total number of bulges (50) and intermediate sections (52) of the inner structure.

10. The filtration device (10) according to claim 8 or 9, **characterized in that** at least one, preferably each, filtrate guiding channel (48a, 48b) of the upper side structure (28) is located opposite a filtrate guiding channel (48a, 48b) of the lower side structure (30), the opposite filtrate guiding channels (48a, 48b) being interconnected by a plurality of through openings (54) allowing filtrate to pass from one filtrate guiding channel (48a, 48b) to the opposite filtrate guiding channel (48a, 48b).

11. The filtration device (10) according to any of claims 4 to 10, **characterized in that** on each side of the filter support (16) only the filtrate guiding channels (48a) leading to the intermediate sections (52) are fluidically connected with the drain hole (26) **in that** each of these filtrate guiding channels (48a) enters into the material of the filter support (16) in front of the inner sealing area (34) and opens at the respective bulge (50) on the opposite side of the filter support (16) into an open space adjoining the drain hole (26).

12. The filtration device (10) according to any of the preceding claims, **characterized in that** outer sealing areas (36) are formed on the upper and lower sides of the filter support (16), the outer sealing areas (36) surrounding the filtrate guiding channels (48a, 48b) and being sealed to the upper and lower filters (40, 42), respectively.

13. The filtration device (10) according to any of the preceding claims, **characterized in that** the housing includes continuous outer welding areas (38) formed on the upper and lower sides of each filter support (16), the outer welding area (38) surrounding the outer sealing area (36) and being welded in a fluid-tight manner to a corresponding outer welding area (38) of a neighboring filter support (16) or to a corresponding outer welding area (38) of the top cover (12) or to a corresponding outer welding area (38) of the bottom cover (14).

14. The filtration device (10) according to any of claims 1 to 12, **characterized in that** the housing (62) includes an annular wall (64) surrounding at least the filter supports (16).

15. The filtration device (10) according to any of the preceding claims, **characterized in that** the bottom cover (14) is equipped with a flat bottom filter (44) having a filtrate side and an opposite unfiltrate side;
the bottom filter (44) being sealed to an upper side of the bottom cover (14) with the filtrate side facing the bottom cover (14) and the unfiltrate side facing away from the bottom cover (14);
a filtrate flow path leading from the filtrate side of the bottom filter (44) to the filtrate outlet (22) of the bottom cover (14), preferably via at least one filtrate guiding channel (48a, 48b) formed on the upper side of the bottom cover (14).

16. The filtration device (10) according to claim 15, **characterized in that** the filtration device (10) does not comprise any filter support (16), the top cover (12) being directly welded to the bottom cover (14).

17. A filter support (16) for use in a filtration device (10), the filter support (16) being adapted to be equipped with a flat upper filter (40) and a flat lower filter (42), the upper and lower filters (40, 42) being shaped identically;
a filtrate flow path leading from the filtrate sides of the upper and lower filters (40, 42) to the filtrate outlet (22) of the bottom cover (14) via at least one filtrate guiding channel (48a, 48b) formed on the upper side of the filter support (16) and at least one filtrate guiding channel (48a, 48b) formed on the lower side of the filter support (16).
